# EUROPEAN PATENT APPLICATION

(11) **EP 3 327 029 A1**
(43) Date of publication of application: **30.05.2018**
(21) Application number: 16827817.4
(22) Date of filing: 21.07.2016
(51) Int. Cl.: C07K 14/195, C07K 1/22, C07K 17/00, C07K 19/00, C12N 1/15, C12N 1/19, C12N 1/21, C12N 5/10, C12N 15/09, C12P 21/08

(54) **ANTIBODY-BINDING PROTEIN HAVING REDUCED ANTIBODY-BINDING CAPACITY IN ACIDIC pH REGIONS**

(30) Priority: 22.07.2015 JP 2015145004
(71) Applicant: Kaneka Corporation, Osaka-shi, Osaka 530-8288 (JP)
(72) Inventor: NISHIHACHIJYO, Masakatsu, Takasago-shi Hyogo 676-8688 (JP); NAKANO, Yoshiyuki, Takasago-shi Hyogo 676-8688 (JP); KONOIKE, Fuminori, Takasago-shi Hyogo 676-8688 (JP); TAKANO, Masayuki, Takasago-shi Hyogo 676-8688 (JP); YOSHIDA, Shinichi, Takasago-shi Hyogo 676-8688 (JP); MINAKUCHI, Kazunobu, Takasago-shi Hyogo 676-8688 (JP)
(74) Representative: Hoffmann Eitle
(86) International application number: PCT/JP2016/071356
(87) International publication number: WO 2017/014257

(57) **Abstract**

The present invention provides a Protein A ligand that has a reduced antibody-binding capacity in an acidic pH range when the ligand is immobilized on a carrier to prepare an affinity separation matrix. The present invention provides a protein containing an amino acid sequence derived from any of the E, D, A, B, and C domains of Protein A of SEQ ID NOs: 1 to 5 in which a hydrophobic amino acid residue in the Fc binding site is substituted by a different hydrophobic amino acid residue or polar uncharged amino acid residue, wherein the protein has a reduced antibody-binding capacity in an acidic pH range as compared to before the substitution.

## Description

### TECHNICAL FIELD

The present invention relates to an antibody-binding protein having a reduced antibody-binding capacity in an acidic pH range.

### BACKGROUND ART

Antibodies function to specifically bind to substances called antigens and to detoxify and remove antigen-containing factors with the cooperation of other biomolecules and cells. The term "antibody" was coined to emphasize such an antigen-binding function, and is also referred to as "immunoglobulin (Ig)" as a chemical name.

Recent developments in genetic engineering, protein engineering, and cell technology have accelerated the development of so-called antibody drugs that utilize the functions of antibodies. Since the antibody drugs more specifically act on target molecules than conventional drugs, they are expected to produce high therapeutic effects while reducing side effects. In fact, they contribute to amelioration of various disease states.

The quality of antibody drugs is considered to largely depend on their purity as compared to other recombinant protein drugs because the antibody drugs are administered in large doses to the body. In order to produce high purity antibodies, techniques using adsorbing materials containing ligand molecules which specifically bind to antibodies (e.g. affinity chromatography) are commonly employed.

The antibody drugs developed so far are generally monoclonal antibodies, which are massively produced by, for example, recombinant cell culture techniques. The "monoclonal antibodies" refer to antibodies that are produced by clones of a single antibody-producing cell. Almost all antibody drugs currently available on the market are classified into immunoglobulin G (IgG) subclasses based on their molecular structure. One well-known example of immunoglobulin-binding proteins having affinity for IgG antibodies is Protein A. Protein A is a cell wall protein produced by the gram-positive bacterium *Staphylococcus aureus* and contains a signal sequence S, five immunoglobulin-binding domains (E domain, D domain, A domain, B domain, and C domain) and a cell wall-anchoring domain known as XM region (Non-Patent Literature 1). The initial purification step (capture step) in antibody drug production processes usually employs an affinity chromatography column where Protein A is immobilized as a ligand on a water-insoluble carrier (hereinafter referred to as Protein A column) (Non-Patent Literatures 1, 2, and 3).

Various techniques for improving the performance of Protein A columns have been developed. Technical developments have also been made in ligands. Initially, wild-type Protein A has been used as a ligand, but recombinant Protein A altered by protein engineering has also appeared as a ligand in many techniques for improving the column performance.

Typical examples of such recombinant Protein A include a recombinant Protein A without the XM region that does not have immunoglobulin-binding activity (rProtein A Sepharose (registered trademark) available from GE Healthcare, Japan). Currently, columns containing as a ligand the recombinant Protein A without the XM region are widely used for industrial purposes because these columns advantageously suppress non-specific adsorption of proteins as compared to conventional ones.

Other inventions have been disclosed wherein a recombinant Protein A in which a single Cys mutation (Patent Literature 1) or a plurality of Lys mutations (Patent Literature 2) is/are introduced into Protein A is used as a ligand. These techniques are effective for immobilization onto water-insoluble carriers and advantageous in terms of capacity to bind antibodies to columns and reduction in leakage of immobilized ligands.

Still other well-known techniques use, as an engineered recombinant Protein A ligand, an engineered domain produced by introducing a mutation into the B domain (this engineered domain is referred to as Z domain) (Non-Patent Literatures 1 and 4 and Patent Literature 3). The Z domain is an engineered B domain in which a mutation is introduced to substitute Gly at position 29 by Ala. In the Z domain, another mutation is simultaneously introduced to substitute Ala at position 1 of the B domain by Val. This mutation is intended to facilitate the genetic engineering preparation of a gene encoding multiple domains linked together and does not affect the domain functions (e.g., a variant produced by substituting Val at position 1 of the Z domain by Ala is used in an example of Patent Literature 4).

The Z domain is known to be more alkali resistant than the B domain and can be advantageously reused in columns by washing with an alkali solution having high sterilizing and washing effects. Ligands based on the Z domain have been devised in which Asn is substituted by another amino acid to impart higher alkali resistance (Patent Literatures 5 and 6), and these ligands are also already used for industrial purposes.

As described above, it is widely appreciated that introducing a substitution of Ala for Gly at position 29 into an immunoglobulin-binding domain (E, D, A, B, or C domain) of Protein A is useful. In fact, the "G29A" mutation was publicly disclosed in 1987, and is also used in prior techniques related to engineered Protein A developed afterwards (Patent Literatures 2, 4, and 6).

Another feature of the Z domain is its reduced ability to bind to the Fab regions of immunoglobulins (Non-Patent Literature 5). This feature advantageously facilitates dissociation of antibodies in the process of dissociating the bound antibodies using acid (Non-Patent Literature 1 and Patent Literature 7). As the antibodies readily dissociate, an eluate having a higher antibody concentration can be recovered using a smaller volume of eluent. In recent antibody drug production processes, the cell culture volume exceeds 10,000 liters per batch, and the antibody expression level has been improved up to nearly 10 g/L in the past few years (Non-Patent Literature 6). This inevitably requires scaling up the throughput of the downstream purification processes, and there is a very large need for improved techniques to recover an eluate having a higher antibody concentration using a smaller volume of eluent.

In addition to the Z domain, engineered Protein A ligands have also been studied based on the C domain of Protein A (Patent Literature 4). These ligands characteristically take advantage of the inherently high alkali resistance of the wild-type C domain and have been receiving attention as new base domains alternative to the Z domain based on the B domain. However, results of studies on the C domain have revealed that the C domain disadvantageously has difficulty in dissociating antibodies in the process of dissociating the antibodies bound to the C domain using acid. As taught in Non-Patent Literature 2 and Patent Literature 4, the C domain has strong ability to bind to the Fab regions of immunoglobulins, and this feature presumably makes it difficult to dissociate the antibodies using acid. In order to ameliorate this drawback, antibody acid dissociation properties were studied on a C domain variant containing a substitution of Gly at position 29 by Ala. As a result, the C domain variant tended to easily dissociate antibodies as compared to the wild-type C domain, but not to a sufficient extent. It is known that antibodies form aggregates or exhibit a decrease in activity at low pH. These phenomena may not only impose load on the purification step in antibody production (an increase in the number of steps or a decrease in yield) but also may result in serious pharmaceutical side effects. Thus, there is a need for a Protein A chromatographic carrier that allows elution at higher pH. Known mutations associated with improvement in antibody acid dissociation properties include a substitution of Ser at position 33, a substitution of His at position 18, and substitutions of His for various amino acid residues (Patent Literatures 8, 9, and 10).

Meanwhile, numerous studies have been made on mutagenesis of Protein A. Amino acid substitution mutagenesis of antibody sites, especially Fc binding sites, of Protein A may induce a significant decrease in antibody binding activity. In particular, hydrophobic amino acid residues such as Phe at position 13, Leu at position 17, and Ile at position 31 are considered to be essential for binding to Fc (Non-Patent Literatures 7 and 8).

### CITATION LIST

### PATENT LITERATURE

Patent Literature 1: U.S. Patent No. 6399750
Patent Literature 2: JP 2007-252368 A
Patent Literature 3: U.S. Patent No. 5143844
Patent Literature 4: JP 2006-304633 A
Patent Literature 5: European Patent No. 1123389
Patent Literature 6: WO 03/080655
Patent Literature 7: U.S. Patent Application No. 2006/0194950
Patent Literature 8: WO 2011/118699
Patent Literature 9: WO 2012/087231
Patent Literature 10: WO 2012/165544

### NON PATENT LITERATURE

Non-Patent Literature 1: Hober S. et al., "J. Chromatogr. B", 2007, Vol. 848, pp. 40-47
Non-Patent Literature 2: Low D. et al., "J. Chromatogr. B", 2007, Vol. 848, pp. 48-63
Non-Patent Literature 3: Roque A. C. A. et al., "J. Chromatogr. A", 2007, Vol. 1160, pp. 44-55
Non-Patent Literature 4: Nilsson B. et al., "Protein Engineering", 1987, Vol. 1, pp. 107-113
Non-Patent Literature 5: Jansson B. et al., "FEMS Immunology and Medical Microbiology", 1998, Vol. 20, pp. 69-78
Non-Patent Literature 6: Junichi Inagawa et al., "Separation process engineering", 2008, Vol. 38, pp. 201-207
Non-Patent Literature 7: O'Seaghdha M. et al., "FEBS J", 2006, Vol. 273, pp. 4831-4841
Non-Patent Literature 8: Tsukamoto M. et al., "J. Biol. Eng.", 2014, Vol. 8, p. 15

### SUMMARY OF INVENTION

### TECHNICAL PROBLEM

The present invention aims to provide a Protein A ligand that has a reduced antibody-binding capacity in an acidic pH range when the ligand is immobilized on a carrier to prepare an affinity separation matrix.

### SOLUTION TO PROBLEM

The present inventors compared and examined the activities of numerous recombinant Protein A variants containing amino acid substitution mutations. As a result, they found that the above problem can be solved by substituting a hydrophobic amino acid residue in an Fc binding site by a different hydrophobic amino acid residue or polar uncharged amino acid residue. This finding has led to the completion of the present invention.

Specifically, the present invention relates to a protein, containing an amino acid sequence derived from any of the E, D, A, B, and C domains of Protein A of SEQ ID NOs: 1 to 5 in which a hydrophobic amino acid residue in an Fc binding site is substituted by a different hydrophobic amino acid residue or polar uncharged amino acid residue, wherein the protein has a reduced antibody-binding capacity in an acidic pH range as compared to before the substitution.

The hydrophobic amino acid residue in the Fc binding site is preferably Phe corresponding to position 5, Phe corresponding to position 13, Leu corresponding to position 17, or Ile corresponding to position 31 of the C domain.

The different hydrophobic amino acid residue is preferably Gly, Ala, Val, Leu, Ile, Met, Phe, or Trp.

The polar uncharged amino acid residue is preferably Ser, Thr, Gln, Asn, Tyr, or Cys.

Preferably, at least 90% of the following amino acid residues are retained: Gln-9, Gln-10, Tyr-14, Pro-20, Asn-21, Leu-22, Gln-26, Arg-27, Phe-30, Leu-34, Pro-38, Ser-39, Leu-45, Leu-51, Asn-52, Gln-55, and Pro-57, wherein the residue numbers indicated are for the C domain.

Preferably, an amino acid corresponding to position 40 of the C domain is an amino acid other than Val.

The amino acid sequence preferably further contains a substitution of a basic amino acid residue for a hydrophobic amino acid residue, an acidic amino acid residue, or a polar uncharged amino acid residue.

The present invention also relates to a multi-domain protein, obtained by linking at least two proteins mentioned above.

The present invention also relates to a DNA, encoding the protein.

The present invention also relates to a vector, containing the DNA.

The present invention also relates to a transformant, produced by transforming a host cell with the vector.

The present invention also relates to a method for producing the protein, the method including using the transformant, or a cell-free protein synthesis system including the DNA.

The present invention also relates to an affinity separation matrix, including the protein as an affinity ligand immobilized on a carrier made of a water-insoluble base material.

The affinity separation matrix preferably binds to a protein containing an immunoglobulin Fc region.

The protein containing an immunoglobulin Fc region is preferably an immunoglobulin G or an immunoglobulin G derivative.

The present invention also relates to a method for preparing the affinity separation matrix, the method including immobilizing the protein as an affinity ligand onto a carrier made of a water-insoluble base material.

The present invention also relates to a method for purifying a protein containing an immunoglobulin Fc region, the method including adsorbing a protein containing an immunoglobulin Fc region onto the affinity separation matrix.

The method preferably includes the following steps (a) and (b): (a) adsorbing a liquid containing a protein containing an immunoglobulin Fc region onto the affinity separation matrix; and (b) bringing an eluent having a pH of 3.5 or higher into contact with the affinity separation matrix to elute the protein containing an immunoglobulin Fc region.

The eluted protein containing an immunoglobulin Fc region preferably contains a reduced amount of host cell proteins and/or aggregates of the protein containing an immunoglobulin Fc region.

### ADVANTAGEOUS EFFECTS OF INVENTION

When the protein of the present invention is immobilized as an affinity ligand on a carrier to prepare an affinity separation matrix, the affinity separation matrix has a reduced antibody-binding capacity in an acidic pH range. This permits elution of antibodies at higher pH than in the prior art.

### BRIEF DESCRIPTION OF DRAWINGS

Fig. 1 is a table for comparison of the sequences of the E, D, A, B, and C domains of Protein A of *Staphylococcus* sp.
Fig. 2 shows the results of an elution test using a C-G29A.2d affinity separation matrix.
Fig. 3 shows the results of an elution test in Example 8 using an engineered C-G29A.2d affinity separation matrix.

### DESCRIPTION OF EMBODIMENTS

The protein of the present invention is characterized in that: it contains an amino acid sequence derived from any of the E, D, A, B, and C domains of Protein A of SEQ ID NOs: 1 to 5 in which a hydrophobic amino acid residue in the Fc binding site is substituted by a different hydrophobic amino acid residue or polar uncharged amino acid residue; and it has a reduced antibody-binding capacity in an acidic pH range as compared to before the substitution.

Protein A is a protein including the immunoglobulin-binding E, D, A, B, and C domains. The E, D, A, B, and C domains are immunoglobulin-binding domains capable of binding to regions other than the complementarity determining regions (CDRs) of immunoglobulins. Each of these domains has activity to bind to the Fc and Fab regions of immunoglobulins and particularly to the Fv regions of the Fab regions. In the present invention, the Protein A may be derived from any source, but is preferably derived from *Staphylococcus* species.

The term "protein" is intended to include any molecule having a polypeptide structure and also encompass fragmentized polypeptide chains and polypeptide chains linked by peptide bonds. The term "domain" refers to a higher-order protein structural unit having a sequence that consists of several tens to hundreds of amino acid residues, enough to fulfill a certain physicochemical or biochemical function.

The domain-derived amino acid sequence means an amino acid sequence before the amino acid substitution. The domain-derived amino acid sequence is not limited only to the wild-type amino acid sequences of the E, D, A, B, and C domains of Protein A, and may include any amino acid sequence partially engineered by amino acid substitution, insertion, deletion, or chemical modification, provided that it forms a protein having the ability to bind to an Fc region. Examples of the domain-derived amino acid sequence include the amino acid sequences of the E, D, A, B, and C domains of *Staphylococcus* Protein A of SEQ ID NOs: 1 to 5. Examples also include proteins having amino acid sequences obtained by introducing a substitution of Ala for Gly corresponding to position 29 of the C domain into the E, D, A, B, and C domains of Protein A. In addition, the Z domain produced by introducing A1V and G29A mutations into the B domain corresponds to the domain-derived amino acid sequence because it also has the ability to bind to an Fc region. The domain-derived amino acid sequence is preferably a domain having high chemical stability or a variant thereof.

The domain-derived amino acid sequence has the ability to bind to an Fc region. The domain-derived amino acid sequence preferably has a sequence identity of 85% or higher, more preferably 90% or higher, still more preferably 95% or higher, to any of the E, D, A, B, and C domains of Protein A of SEQ ID NOs: 1 to 5.

The protein of the present invention contains an amino acid sequence derived from the E, D, A, B, or C domain of Protein A in which a hydrophobic amino acid residue in the Fc binding site is substituted by a different hydrophobic amino acid residue or polar uncharged amino acid residue.

The amino acid substitution means a mutation which deletes the original amino acid and adds a different type of amino acid to the same position. It should be noted that amino acid substitutions are denoted herein with the code for the wild-type or non-mutated type amino acid, followed by the position number of the substitution, followed by the code for changed amino acid. For example, a substitution of Ala for Gly at position 29 is represented by G29A.

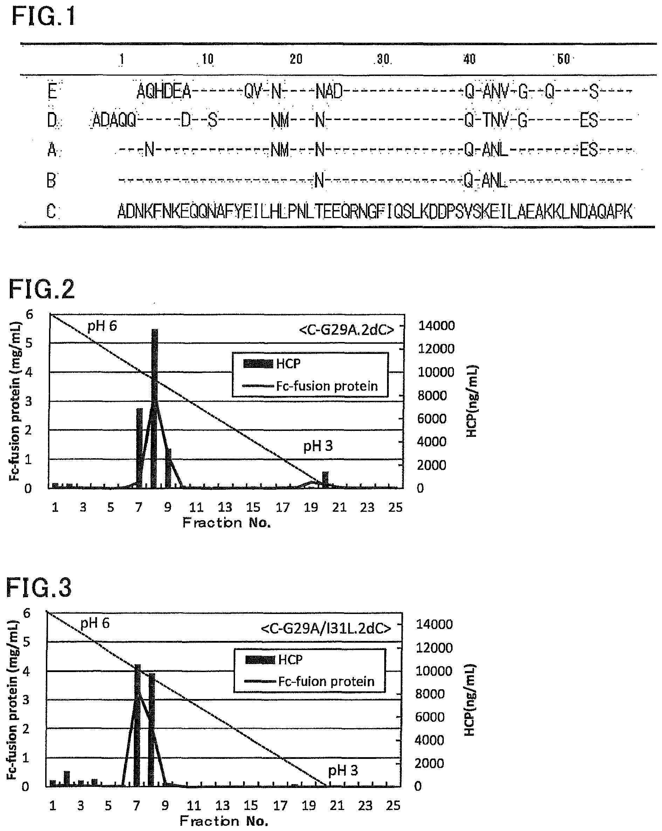

Examples of the hydrophobic amino acid residue in the Fc binding site include Phe corresponding to position 5, Phe corresponding to position 13, Leu corresponding to position 17, and Ile corresponding to position 31 of the C domain. The term "corresponding" means that they are in the same column when the E, D, A, B, and C domains of Protein A are aligned as shown in Fig. 1.

Examples of the different hydrophobic amino acid residue used for substitution include Gly, Ala, Val, Leu, Ile, Met, Phe, and Trp. Preferred among these are Ala, Val, Leu, Ile, and Phe. The term "different hydrophobic amino acid residue" refers to a hydrophobic amino acid residue different from the original hydrophobic amino acid residue to be substituted. For example, when the original amino acid residue to be substituted is Phe corresponding to position 5 or 13 of the C domain, the different hydrophobic amino acid residue may be any of the above-mentioned different amino acid residues other than Phe. When the original amino acid residue to be substituted is Leu corresponding to position 17 of the C domain, the different hydrophobic amino acid residue may be any of the above-mentioned different amino acid residues other than Leu. When the original amino acid residue to be substituted is Ile corresponding to position 31 of the C domain, the different hydrophobic amino acid residue may be any of the above-mentioned different amino acid residues other than Ile.

Examples of the polar uncharged amino acid residue used for substitution include Ser, Thr, Gln, Asn, Tyr, and Cys. Preferred among these are Ser, Thr, Gln, Asn, and Tyr.

More specific substitution embodiments include a substitution of Ala or Tyr for Phe corresponding to position 5 of the C domain, a substitution of Tyr for Phe corresponding to position 13 of the C domain, a substitution of Ile, Val, or Thr for Leu corresponding to position 17 of the C domain, and a substitution of Leu, Ser, Thr, or Val for Ile corresponding to position 31 of the C domain. Preferred among these are F5A and F5Y in the C domain, F13Y in the C domain, L17I, L17V, and L17T in the C domain, I31L, I31S, I31T, and I31V in the C domain, and I31L and I31T in the B domain.

The number of amino acid substitutions in the Fc binding site is not particularly limited, provided that the antibody-binding capacity in an acidic pH range is reduced as compared to before substitution. Yet, the number is preferably 4 or less, more preferably 2 or less, in order to maintain the conformation of the protein before mutagenesis and to maintain the antibody-binding capacity in a neutral range. Embodiments containing two amino acid substitutions may be obtained, for example, by substitution of L17I and I31L in the C domain or by similarly substituting amino acids at positions corresponding to positions 17 and 13 of the C domain in the E, D, A, or B domain.

As long as the antibody-binding capacity in an acidic pH range is reduced as compared to before substitution, the protein may contain any amino acid substitution, in addition to the substitution of a hydrophobic amino acid residue in the Fc binding site by a different hydrophobic amino acid residue or polar uncharged amino acid residue. Examples of such amino acid substitutions include G29A substitution in the C domain. Examples also include similar substitutions of an amino acid at a position corresponding to positon 29 of the C domain in the E, D, A, and B domains.

Moreover, the any amino acid substitution may be a substitution of Val corresponding to position 40 of the C domain by an amino acid residue other than Val. Specific examples of such substitutions include V40Q in the C domain. Other examples include a substitution of a hydrophobic amino acid residue, an acidic amino acid residue, or a polar uncharged amino acid residue by a basic amino acid residue. Specific examples of such substitutions include A12R, L19R, L22R, Q26R, Q32R, S33H, and V40H in the C domain and similar substitutions in the E, D, A, B, and B domains.

The amino acid sequence derived from any of the E, D, A, B, and C domains of Protein A of SEQ ID NOs: 1 to 5 in which a hydrophobic amino acid residue in the Fc binding site is substituted by a different hydrophobic amino acid residue or polar uncharged amino acid residue preferably has a sequence identity of 85% or higher, more preferably 90% or higher, still more preferably 95% or higher, to any of the E, D, A, B, and C domains of Protein A of SEQ ID NOs: 1 to 5.

In the protein of the present invention, preferably at least 90%, more preferably at least 95%, of the following amino acid residues are retained: Gln-9, Gln-10, Tyr-14, Pro-20, Asn-21, Leu-22, Gln-26, Arg-27, Phe-30, Leu-34, Pro-38, Ser-39, Leu-45, Leu-51, Asn-52, Gln-55, and Pro-57 (the residue numbers indicated are for the C domain).

The protein of the present invention is characterized by having a reduced antibody-binding capacity in an acidic pH range as compared to before substitution. The acidic pH range may be a weakly acidic range, specifically with a pH in the range of 3 to 6.

The antibody-binding capacity in the acidic range can be evaluated by a pH gradient elution test using IgG Sepharose (Example 1), measurement of the antibody-binding capacity in an acidic pH range using an intermolecular interaction analyzer, or an antibody elution test using an affinity separation matrix with an immobilized ligand (Example 5). For example, in the case of a pH gradient elution test using IgG Sepharose, a variant that has a reduced antibody-binding capacity in an acidic range as compared to the non-mutated protein (e.g. C-G29A.2d) elutes at higher pH. When the elution pH calculated from the top of the elution peak of the non-mutated protein is taken as reference, the elution pH of the variant is preferably higher than the reference by 0.05 or more, more preferably by 0.1 or more. Also, in the case of an antibody elution test using an affinity separation matrix with an immobilized ligand, a comparison is made between the antibody recovery rates of an affinity separation matrix with an immobilized non-mutated ligand (e.g. C-G29A.2d) and an affinity separation matrix with an immobilized variant thereof after an antibody is eluted using a high pH eluent (e.g., pH 4). The antibody recovery rate of the affinity separation matrix with the immobilized variant is preferably higher than that of the affinity separation matrix with the immobilized non-mutated ligand by 1% or higher, more preferably by 5% or higher.

The protein of the present invention may be a protein consisting only of a single domain in which the amino acid substitution is introduced, or a multi-domain protein obtained by linking at least two domains in which the amino acid substitution is introduced.

In the case of a multi-domain protein, the proteins to be linked may be the same domain-derived proteins (i.e., a homopolymer such as a homodimer or homotrimer) or different domain-derived proteins (i.e., a heteropolymer such as a heterodimer or heterotrimer). The number of proteins linked is preferably 2 or more, and more preferably 2 to 10, still more preferably 2 to 6.

In the multi-domain protein, the monomeric proteins or single domains may be linked to each other by, for example, but not limited to: a method that does not use an amino acid residue as a linker; or a method that uses one or more amino acid residues. The number of amino acid residues used for linkage is not particularly limited. The linkage mode and the number of linkages are also not particularly limited, provided that the three-dimensional conformation of the monomeric proteins does not become unstable.

Moreover, the protein of the present invention may include a fusion protein in which the above-described protein or multi-domain protein, as one component, is fused with another protein having a different function. Non-limiting examples of such fusion proteins include those fused with albumin, GST (glutathione S-transferase), or MBP (maltose-binding protein). Expression as a fusion protein with GST or MBP facilitates purification of the protein. Those fused with a nucleic acid (e.g. DNA aptamer), a drug (e.g. antibiotic substance) or a polymer (e.g. polyethylene glycol (PEG)) are also encompassed in the protein of the present invention, provided that they have the same effect as the present invention.

The present invention also relates to a DNA encoding the protein. The DNA may be any DNA having a base sequence that is translated into the amino acid sequence of the protein of the present invention. Such a base sequence can be obtained by common known techniques, such as polymerase chain reaction (hereinafter abbreviated as PCR). Alternatively, it can be synthesized by known chemical synthesis techniques or may be available from DNA libraries. A codon in the base sequence may be replaced with a degenerate codon, and the base sequence is not necessarily the same as the original base sequence, provided that the coding base sequence is translated into the same amino acids.

The DNA of the present invention can be obtained by site-directed mutagenesis of a conventionally known DNA encoding a wild-type or mutated Protein A domain. Site-directed mutagenesis may be performed by, for example, recombinant DNA technology or PCR as follows.

In the case of mutagenesis by recombinant DNA technology, for example, if there are suitable restriction enzyme recognition sequences on both sides of a mutagenesis target site in the gene encoding the protein of the present invention, a cassette mutagenesis method can be used in which these restriction enzyme recognition sites are cleaved with the restriction enzymes to remove a region containing the mutagenesis target site, and a DNA fragment in which only the target site is mutated by chemical synthesis or other methods is then inserted.

In the case of site-directed mutagenesis by PCR, for example, a double primer method can be used in which PCR is performed using a double-stranded plasmid encoding the protein as a template and two synthetic oligo primers containing complementary mutations in the + and - strands.

A DNA encoding the multi-domain protein can be prepared by ligating the desired number of DNAs encoding the monomeric protein (single domain) of the present invention in tandem. For example, the DNA encoding the multi-domain protein may be prepared by a ligation method in which a suitable restriction enzyme site is introduced into a DNA sequence, which is then cleaved with the restriction enzyme into a double-stranded DNA fragment, followed by ligation using a DNA ligase. A single restriction enzyme site or a plurality of different restriction enzyme sites may be introduced. Alternatively, the DNA encoding the multi-domain protein may be prepared by applying any of the mutagenesis methods to a DNA encoding Protein A (e.g., see WO 06/004067). Here, if the base sequences each encoding a monomeric protein in the DNA encoding the multi-domain protein are the same, then homologous recombination may be induced in host cells. For this reason, the ligated DNAs encoding a monomeric protein preferably have 90% or lower, more preferably 85% or lower base sequence identity.

The vector of the present invention includes a base sequence encoding the above-described protein or multi-domain protein, and a promoter that is operably linked to the base sequence to function in a host cell. Typically, the vector can be constructed by linking or inserting the above-described DNA encoding the protein into a vector.

The vector used for insertion of the gene is not particularly limited, provided that it is capable of autonomous replication in a host cell. The vector may be a plasmid DNA or phage DNA. When *Escherichia coli* is used as a host cell, examples of the vector used for insertion of the gene include pQE vectors (QIAGEN), pET vectors (Merck), and pGEX vectors (GE Healthcare, Japan). When *Brevibacillus* is used as a host cell, examples include the known *Bacillus subtilis* vector pUB110 and pHY500 (JP H02-31682 A), pNY700 (JP H04-278091 A), pNU211R2L5 (JP H07-170984 A), pHT210 (JP H06-133782 A), and the shuttle vector pNCMO2 between *Escherichia coli* and *Brevibacillus* (JP 2002-238569 A).

A transformant can be produced by transforming a host cell with the vector. Any host cell may be used. For low-cost mass production, *Escherichia coli, Bacillus subtilis,* and bacteria (eubacteria) of genera including *Brevibacillus, Staphylococcus, Streptococcus, Streptomyces,* and *Corynebacterium* can be suitably used. More preferred are gram-positive bacteria such as *Bacillus subtilis* and bacteria of the genera *Brevibacillus, Staphylococcus, Streptococcus, Streptomyces,* and *Corynebacterium.* Still more preferred are bacteria of the genus *Brevibacillus,* which are known for their application in mass production of Protein A (WO 06/004067).

Examples of the bacteria of the genus *Brevibacillus* include, but are not limited to: *Brevibacillus agri, B. borstelensis, B. brevis, B. centrosporus, B. choshinensis, B. formosus, B. invocatus, B. laterosporus, B. limnophilus*, *B. parabrevis, B. reuszeri,* and *B. thermoruber.* Preferred examples include *Brevibacillus brevis* 47 (JCM6285), *Brevibacillus brevis* 47K (FERM BP-2308), *Brevibacillus brevis* 47-5Q (JCM8970), *Brevibacillus choshinensis* HPD31 (FERM BP-1087), and *Brevibacillus choshinensis* HPD31-OK (FERM BP-4573). Mutants (or derivative strains) such as protease-deficient strains, high-expressing strains, or sporulation-deficient strains of the *Brevibacillus* bacteria may be used for purposes such as improved yield. Specific examples include the protease mutant *Brevibacillus choshinensis* HPD31-OK (JP H06-296485 A) and sporulation-deficient *Brevibacillus choshinensis* HPD31-SP3 (WO 05/045005), which are derived from *Brevibacillus choshinensis* HPD31.

The vector may be introduced into the host cell by, for example, but not limited to: a calcium ion method, an electroporation method, a spheroplast method, a lithium acetate method, an agrobacterium infection method, a particle gun method, or a polyethylene glycol method. Moreover, the obtained gene function may be expressed in the host cell, for example, by incorporating the gene obtained in the present invention into a genome (chromosome).

The transformant, or a cell-free protein synthesis system including the DNA can be used to produce the protein.

In the case where the transformant is used to produce the protein, the transformed cell may be cultured in a medium to produce and accumulate the protein of the present invention in the cultured cells (including the periplasmic space thereof) or in the culture medium (extracellularly), and the desired protein can be collected from the culture.

When the transformed cell is used to produce the protein, the protein may be accumulated within the transformant cell and/or in the periplasmic space thereof. In this case, the accumulation within the cell is advantageous in that the expressed protein can be prevented from oxidation, and there are no side reactions with the medium components. On the other hand, the accumulation in the periplasmic space is advantageous in that decomposition by intracellular proteases can be suppressed. Alternatively, the protein may be produced by secreting the protein extracellularly of the transformant. This does not require cell disruption and extraction steps and is thus advantageous for reducing production costs.

The transformed cell of the present invention can be cultured in a medium according to common methods for culturing host cells. The medium used for culturing the transformant is not particularly limited, provided that it allows for high yield and high efficiency production of the protein. Specifically, carbon and nitrogen sources such as glucose, sucrose, glycerol, polypeptone, meat extracts, yeast extracts, and casamino acids can be used. In addition, the medium is supplemented with inorganic salts such as potassium salts, sodium salts, phosphates, magnesium salts, manganese salts, zinc salts, or iron salts, as necessary. In the case of an auxotrophic host cell, nutritional substances necessary for its growth may be added. Moreover, antibiotics such as penicillin, erythromycin, chloramphenicol, and neomycin may optionally be added.

Furthermore, a variety of known protease inhibitors, phenylmethane sulfonyl fluoride (PMSF), benzamidine, 4-(2-aminoethyl)-benzenesulfonyl fluoride (AEBSF), antipain, chymostatin, leupeptin, pepstatin A, phosphoramidon, aprotinin, and ethylenediaminetetraacetic acid (EDTA), and/or other commercially available protease inhibitors may be added at appropriate concentrations in order to reduce the degradation or molecular-size reduction of the target protein caused by host-derived proteases present inside or outside the cells.

In order to ensure accurate folding of the protein of the present invention, molecular chaperones such as GroEL/ES, Hsp70/DnaK, Hsp90, or Hsp104/ClpB may be used. In this case, for example, they can be allowed to coexist with the protein of the present invention by, for example, co-expression or incorporation into a fusion protein. Other methods for ensuring accurate folding of the protein of the present invention may also be used such as, but not limited to, adding an additive for assisting accurate folding to the medium or culturing at low temperatures.

Examples of media that can be used to culture the transformed cell obtained using *Escherichia coli* as a host include LB medium (1% triptone, 0.5% yeast extract, 1% NaCl) and 2×YT medium (1.6% triptone, 1.0% yeast extract, 0.5% NaCl).

Examples of media that can be used to culture the transformant obtained using *Brevibacillus* as a host include TM medium (1% peptone, 0.5% meat extract, 0.2% yeast extract, 1% glucose, pH 7.0) and 2SL medium (4% peptone, 0.5% yeast extract, 2% glucose, pH 7.2).

The cell is aerobically cultured at a temperature of 15°C to 42°C, preferably 20°C to 37°C, for several hours to several days under aeration and stirring conditions to accumulate the protein of the present invention in the cultured cells (including the periplasmic space thereof) or in the culture medium (extracellularly), followed by recovery of the protein. In some cases, the cell may be cultured anaerobically without air.

In the case where the recombinant protein is secreted, the produced recombinant protein can be recovered after the culture by separating the cultured cells from the supernatant containing the secreted protein by a common separation method such as centrifugation or filtration.

Also in the case where the protein is accumulated in the cultured cells (including the periplasmic space), the protein accumulated in the cells can be recovered, for example, by collecting the cells from the culture medium, e.g. via centrifugation or filtration, followed by disrupting the cells, e.g. via sonication or French press, and/or solubilizing the protein with, for example, a surfactant.

In the case where the protein of the present invention is produced using a cell-free protein synthesis system, the cell-free protein synthesis system is not particularly limited. Examples include synthesis systems derived from procaryotic cells, plant cells, or higher animal cells.

The protein of the present invention can be purified by methods such as affinity chromatography, cation or anion exchange chromatography, and gel filtration chromatography, used alone or in an appropriate combination.

Whether the purified product is the target protein may be confirmed by common techniques such as SDS polyacrylamide gel electrophoresis, N-terminal amino acid sequencing, or Western blot analysis.

An affinity separation matrix can be prepared by immobilizing the thus produced protein as an affinity ligand onto a carrier made of a water-insoluble base material. The term "affinity ligand" means a substance (functional group) that selectively captures (binds to) a target molecule from a mixture of molecules by virtue of a specific affinity between the molecules such as antigen-antibody binding, and refers herein to a protein that specifically binds to an immunoglobulin. The term "ligand" as used alone herein is synonymous with "affinity ligand".

Examples of the carrier made of a water-insoluble base material used in the present invention include inorganic carriers such as glass beads and silica gel; organic carriers such as synthetic polymers (e.g. cross-linked polyvinyl alcohol, cross-linked polyacrylate, cross-linked polyacrylamide, cross-linked polystyrene) and polysaccharides (e.g. crystalline cellulose, cross-linked cellulose, cross-linked agarose, cross-linked dextran); and composite carriers formed by combining these carriers such as organic-organic or organic-inorganic composite carriers. Examples of commercially available products include GCL2000 (porous cellulose gel), Sephacryl S-1000 (prepared by covalently cross-linking allyl dextran with methylene bisacrylamide), Toyopearl (methacrylate carrier), Sepharose CL4B (cross-linked agarose carrier), and Cellufine (cross-linked cellulose carrier), although the water-insoluble carrier used in the present invention is not limited to the carriers listed above.

In view of the purpose and method of using the affinity separation matrix, the water-insoluble carrier used in the present invention should have a large surface area and is preferably a porous material having a large number of fine pores of an appropriate size. The carrier may be in any form such as bead, monolith, fiber, film (including hollow fiber) or other optional forms.

The immobilization of the ligand onto the carrier may be carried out by, for example, conventional coupling methods utilizing an amino, carboxyl, or thiol group on the ligand. such coupling may be accomplished by an immobilization method that includes reacting the carrier with cyanogen bromide, epichlorohydrin, diglycidyl ether, tosyl chloride, tresyl chloride, hydrazine, sodium periodate, or the like to activate the carrier (or introduce a reactive functional group into the carrier surface), and performing a coupling reaction between the carrier and the compound to be immobilized as a ligand; or an immobilization method that includes adding a condensation reagent such as carbodiimide or a reagent having a plurality of functional groups in the molecule such as glutaraldehyde to a system containing the carrier and the compound to be immobilized as a ligand, followed by condensation and cross-linking.

A spacer molecule consisting of a plurality of atoms may be introduced between the ligand and the carrier, or alternatively, the ligand may be directly immobilized onto the carrier. Accordingly, for immobilization, the protein of the present invention may be chemically modified or may incorporate an additional amino acid residue useful for immobilization. Examples of amino acids useful for immobilization include amino acids having in a side chain a functional group useful for a chemical reaction for immobilization, such as Lys which contains an amino group in a side chain, and Cys which contains a thiol group in a side chain. Whatever modification or alteration is made for immobilization, the resulting protein is included within the scope of the present invention as the nature of the present invention is such that the effect provided to the protein by the present invention is also provided to the matrix on which the protein is immobilized as a ligand.

The affinity separation matrix obtained by immobilization of the protein of the present invention is capable of binding to a protein containing an immunoglobulin Fc region due to the activity of the protein of the present invention itself. Accordingly, the protein and the affinity separation matrix of the present invention can be used to separate and purify a protein containing an immunoglobulin Fc region by an affinity column chromatography purification method. The term "protein containing an immunoglobulin Fc region" refers to a protein containing an Fc region portion to which Protein A binds. However, the protein does not have to contain the entire Fc region, provided that Protein A can bind thereto.

Non-limiting examples of the protein containing an immunoglobulin Fc region include immunoglobulin G and immunoglobulin G derivatives.

The term "immunoglobulin G derivative" is a generic term for engineered artificial proteins to which Protein A can bind, and examples include chimeric immunoglobulin G in which the domains of human immunoglobulin G are partially replaced and fused with immunoglobulin G domains of another biological species, humanized immunoglobulin G in which complementarity determining regions (CDRs) of human immunoglobulin G are replaced and fused with antibody CDRs of another biological species, immunoglobulin G in which a sugar chain in the Fc region is molecularly altered, artificial immunoglobulin G in which the Fv and Fc regions of human immunoglobulin G are fused, and fusion proteins in which a useful protein is fused with the Fc region of human immunoglobulin G. Examples of the useful protein include various receptors, cytokines, hormones, and enzymes. Examples of receptors include TNF receptor, VEGF receptor, and the extracellular region of CTLA4. Examples of cytokines include thrombopoietin receptor peptide. Examples of hormones include GLP-1 peptide. Examples of enzymes include blood coagulation factor VIII, blood coagulation factor IX, and phosphatase.

As described earlier, the regions to be bound are broadly specified as Fab regions (particularly Fv regions) and Fc regions. However, since the conformation of antibodies is already known, the proteins to which the protein and the affinity separation matrix of the present invention bind may be ones obtained by further altering (e.g. fragmentizing) the Fab or Fc regions while maintaining the conformation of the regions to which Protein A binds by protein engineering techniques.

The protein containing an immunoglobulin Fc region can be purified by the steps of: bringing the protein containing an immunoglobulin Fc region into contact with the affinity separation matrix containing a ligand immobilized on a carrier to adsorb the protein onto the affinity separation matrix; and bringing an eluent having a pH of 3.0 or higher into contact with the affinity separation matrix to elute the protein containing an immunoglobulin Fc region.

In the first step of the method for purifying the antibody-like protein, the protein containing an immunoglobulin Fc region is brought into contact with the affinity separation matrix containing a ligand immobilized on a carrier to adsorb the protein containing an immunoglobulin Fc region onto the affinity separation matrix. Specifically, a buffer containing the protein containing an immunoglobulin Fc region is adjusted to be neutral, and the resulting solution is passed through an affinity column filled with the affinity separation matrix to adsorb the protein containing an immunoglobulin Fc region. Examples of the buffer include citric acid, 2-(N-morpholino)ethanesulfonic acid (MES), Bis-Tris, N-(2-acetamido)iminodiacetic acid (ADA), piperazine-1,4-bis(2-ethanesulfonic acid) (PIPES), N-(2-acetamido)-2-aminoethanesulfonic acid (ACES), 3-(N-morpholino)-2-hydroxypropanesulfonic acid (MOPSO), N,N-bis(2-hydroxyethyl)-2-aminoethanesulfonic acid (BES), 3-(N-morpholino)propanesulfonic acid (MOPS), N-tris(hydroxymethyl)methyl-2-aminoethanesulfonic acid (TES), 4-(2-hydroxyethyl)-1-piperazineethanesulfonic acid (HEPES), triethanolamine, 3-[4-(2-hydroxyethyl)-1-piperazinyl]propanesulfonic acid (EPPS), Tricine, Tris, glycylglycine, Bicine, N-tris(hydroxymethyl)methyl-3-aminopropanesulfonic acid (TAPS), and Dulbecco's phosphate buffered saline. The pH at which the protein containing an immunoglobulin Fc region is adsorbed onto the affinity separation matrix is preferably 6.5 to 8.5, more preferably 7 to 8. The temperature at which the antibody-like protein is adsorbed onto the affinity separation matrix is preferably 1°C to 40°C, more preferably 4°C to 25°C.

The first step may be followed by passing an appropriate amount of pure buffer through the affinity column to wash the inside of the column. At this point, the desired antibody-like protein remains adsorbed on the affinity separation matrix in the column. The buffer for washing may be the same as the buffer used in the first step.

In the second step of the method for purifying the protein containing an immunoglobulin Fc region, an eluent having a pH of 3.5 or higher is brought into contact with the affinity separation matrix to elute the protein containing an immunoglobulin Fc region. Examples of the eluent include citrate buffer, acetate buffer, phosphate buffer, glycine buffer, formate buffer, propionate buffer, γ-aminobutyrate buffer, and lactate buffer.

The antibody can be recovered as long as the pH of the eluent is 3.0 or higher. Yet, it is suitable to use an eluent having a higher pH which can avoid aggregation of antibodies and a reduction in antibody activity. Specifically, the pH is more preferably 3.5 or higher, still more preferably 3.6 or higher, further more preferably 3.75 or higher, particularly preferably 3.8 or higher, most preferably 3.9 or higher, even most preferably 4.0 or higher. The upper pH limit of the eluent is preferably 6.0.

The elution of the antibody from the affinity separation matrix may also be carried out in a stepwise manner using different pH eluents. Moreover, gradient elution with a pH gradient using two or more eluents with different pH values (e.g. pH 6 and pH 3) is suitable because higher purification can be achieved. Since the affinity separation matrix of the present invention allows elution of the antibody under particularly high pH conditions, the eluents in the gradient elution preferably partially include an eluent having a pH of 4 to 6. A surfactant (such as Tween 20 or Triton-X 100), a chaotropic agent (such as urea or guanidine), or an amino acid (such as arginine) may also be added to the buffer used for adsorption, washing, or elution.

Similarly, the pH in the affinity column filled with the affinity separation matrix at the time of elution of the protein containing an immunoglobulin Fc region is preferably 3.0 or higher, more preferably 3.5 or higher, still more preferably 3.6 or higher, yet still more preferably 3.75 or higher, particularly preferably 3.8 or higher, most preferably 3.9 or higher, even most preferably 4.0 or higher. When elution is performed at a pH of 3.0 or higher, damage to the antibody can be reduced (Ghose S. et al., Biotechnology and bioengineering, 2005, vol. 92, No. 6). The upper limit of the pH in the affinity column filled with the affinity separation matrix at the time of elution of the protein containing an immunoglobulin Fc region is preferably 6.0. According to the purification method of the present invention, the protein containing an immunoglobulin Fc region can be dissociated under acidic elution conditions closer to neutral, so that a sharper elution peak profile can be obtained when the protein containing an immunoglobulin Fc region is eluted under acidic conditions. Due to the sharper chromatographic elution peak profile, a smaller volume of eluent can be used to recover an eluate having a higher antibody concentration.

The temperature at which the protein containing an immunoglobulin Fc region is eluted is preferably 1°C to 40°C, more preferably 4°C to 25°C.

The recovery rate of the protein containing an immunoglobulin Fc region recovered by the purification method of the present invention is preferably 90% or higher, more preferably 95% or higher. The recovery rate may be calculated using the following equation, for example. Recovery rate (%) = [(Concentration (mg/mL) of eluted protein containing immunoglobulin Fc region) × (Volume (ml) of eluted liquid)] ÷ [(Concentration (mg/mL) of loaded protein containing immunoglobulin Fc region) × (Volume (ml) of loaded liquid)] × 100

According to the purification method of the present invention, it is possible to reduce contamination of host cell proteins (HCP) for expressing the protein containing an immunoglobulin Fc region. It is also possible to reduce contamination of aggregates of the protein containing an immunoglobulin Fc region. The contamination of these proteins may increase the load on the purification step in antibody production (an increase in the number of steps or a decrease in yield), and may also result in serious pharmaceutical side effects due to the impurity proteins. In contrast, the purification method of the present invention can avoid these problems.

The affinity separation matrix of the present invention is effective in separating the protein containing an immunoglobulin Fc region from host cell proteins. The host cell from which the host cell proteins originate is a cell capable of expressing the protein containing an immunoglobulin Fc region, such as particularly a CHO cell or *Escherichia coli,* for which gene recombination techniques have been established. Such host cell proteins can be quantified using commercially available immunoassay kits. For example, CHO cell proteins may be quantified with CHO HCP ELISA kit (Cygnus).

The affinity separation matrix of the present invention is effective in purifying the non-aggregated protein containing an immunoglobulin Fc region from a solution containing aggregates of the protein containing an immunoglobulin Fc region, e.g. in an amount of at least 1%, 5%, or 10% of the total amount of the protein containing an immunoglobulin Fc region in the eluate, to remove the aggregates. The amount of the aggregates may be analyzed and quantified by, for example, gel filtration chromatography.

The affinity separation matrix of the present invention can be reused by passing through it a pure buffer having an appropriate strong acidity or strong alkalinity which does not completely impair the functions of the ligand compound and the carrier base material (or optionally a solution containing an appropriate modifying agent or an organic solvent) for washing.

The affinity of the protein and the affinity separation matrix of the present invention for the protein containing an immunoglobulin Fc region may be tested using, for example, biosensors such as Biacore system (GE Healthcare, Japan) based on the principle of surface plasmon resonance. When the affinity of the protein of the present invention for the immunoglobulin is measured as an affinity for a human immunoglobulin G preparation using the Biacore system, which will be described later, the association constant (K_{A}) is preferably 10⁶ (M⁻¹) or higher, more preferably 10⁷ (M⁻¹) or higher.

The measurement may be carried out under any conditions that allow detection of a binding signal corresponding to the binding of the protein of the present invention to the immunoglobulin Fc region. The affinity can be easily evaluated at a (constant) temperature of 20°C to 40°C and a neutral pH of 6 to 8.

Examples of immunoglobulin molecules that can be used as binding partners include gammaglobulin "Nichiyaku" (human immunoglobulin G, Nihon Pharmaceutical Co. Ltd.) which is a polyclonal antibody, and commercially available pharmaceutical monoclonal antibodies.

A skilled person can easily evaluate the difference in affinity by preparing and analyzing sensorgrams of binding to the same immunoglobulin molecule under the same measurement conditions, and using the obtained binding parameters to compare the proteins before and after mutagenesis.

Examples of binding parameters that can be used include association constant (K_{A}) and dissociation constant (K_{D}) (Nagata et al., "Real-time analysis of biomolecular interactions", Springer-Verlag Tokyo, 1998, page 41). The association constant between each domain variant according to the present invention and Fab may be determined in an experimental system using Biacore system in which an Fab fragment of an immunoglobulin of the VH3 subfamily is immobilized on a sensor chip, and each domain variant is added to a flow channel at a temperature of 25°C and a pH of 7.4. Although the association constant may also be described as affinity constant in some documents, the definitions of these terms are essentially the same.

### EXAMPLES

The present invention is more specifically described below with reference to examples, but the present invention is not limited to these examples. In the examples, operations such as recombinant DNA production and engineering were performed in accordance with the following textbooks, unless otherwise noted: (1) T. Maniatis, E. F. Fritsch, and J. Sambrook, "Molecular Cloning/A Laboratory Manual", second edition (1989), Cold Spring Harbor Laboratory (USA); and (2) Masami Muramatsu, "Lab Manual for Genetic Engineering", third edition (1996), Maruzen Co., Ltd. The materials such as reagents and restriction enzymes used in the examples were commercially available products, unless otherwise specified.

Proteins obtained in the examples are represented by "an alphabetical letter identifying the domain - an introduced mutation (wild for the wild type)". For example, the wild-type C domain of Protein A is represented by "C-wild", and a C domain variant containing G29E mutation is represented by "C-G29E". Variants containing two mutations together are represented by indicating both with a slash. For example, a C domain variant containing G29E and S13L mutations is represented by "C-G29E/S13L". Proteins consisting of a plurality of single domains linked together are represented by adding a period (.) followed by the number of linked domains followed by "d". For example, a protein consisting of five linked C domain variants containing G29E and S13L mutations is represented by "C-G29E/S13L.5d".

### (Example 1) Evaluation of antibody-binding capacity of C domain variant using IgG-immobilized carrier

The total synthesis of artificially synthesized genes of engineered C-G29A.2d variants was outsourced to Eurofins Genomics K.K. These genes were synthesized by introducing amino acid substitution mutations as shown in Table 1 into a DNA (SEQ ID NO: 7) obtained by adding PstI and XbaI recognition sites to the 5' and 3' ends, respectively, of a DNA encoding C-G29A.2d (SEQ ID NO: 6) containing G29A mutation in the C domain of Protein A. They were subcloned into expression plasmids, which were then digested with the restriction enzymes PstI and XbaI (Takara Bio, Inc.), and each of the obtained DNA fragments was ligated to a *Brevibacillus* expression vector pNCMO2 (Takara Bio, Inc.) digested with the same restriction enzymes to construct expression plasmids in which a DNA encoding the amino acid sequence of each engineered C-G29A.2d was inserted into a *Brevibacillus* expression vector pNCMO2. The plasmids were prepared using *Escherichia coli* JM109.

*Brevibacillus choshinensis* SP3 (Takara Bio, Inc.) was transformed with each of the obtained plasmids, and the recombinant cells capable of secreting each engineered C-G29A.2d were grown. These recombinant cells were cultured with shaking for three days at 30°C in 30 mL of A medium (3.0% polypeptone, 0.5% yeast extract, 3% glucose, 0.01% magnesium sulfate, 0.001% iron sulfate, 0.001% manganese chloride, 0.0001% zinc chloride) containing 60 µg/mL of neomycin.

The amino acid sequences of C-F5A/G29A.2d, C-F5Y/G29A.2d, C-F5G/G29A.2d, C-F5M/G29A.2d, C-F13Y/G29A.2d, C-F13W/G29A.2d, C-L17I/G29A.2d, C-L17I/G29A/I31L.2d, C-L17T/G29A.2d, C-L17V/G29A.2d, C-G29A/I31L.2d, C-G29A/I31F.2d, C-G29A/I31N.2d, C-G29A/I31L/S33H.2d, C-G29A/I31L/V40Q.2d, C-G29A/I31S.2d, C-G29A/I31T.2d, and C-G29A/I31V.2d expressed as above are shown in SEQ ID NOs: 10 to 27, respectively, in the Sequence Listing.

After the culture, the cells were removed from the culture medium by centrifugation (15,000 rpm at 25°C for 5 min). Subsequently, the concentration of each engineered C-G29A.2d in the culture supernatant was measured by high performance liquid chromatography. An elution test was performed on each engineered C-G29A.2d or C-G29A.2d in the culture supernatant using an IgG-immobilized carrier under the following conditions.

### <Conditions for elution test using IgG-immobilized carrier>

Carrier: IgG Sehparose FF (GE Healthcare)
Column: Omnifit column (Diba Industries); column diameter:
0.66 cm; bed height: 6.4 cm; column volume: 2.19 mL
Flow rate: 0.8 mL/min; contact time: 2.7 min
Loading volume: 470 µL (ligand concentration: 1.3 mg/mL)
Equilibration buffer: 50 mM Tris-HCl, 150 mM NaCl buffer, pH 7.5
Elution conditions: 50 mM citrate buffer (pH 6.0), followed by 50 mM citrate buffer (pH 3.0) (20 CV)

The difference between the elution pHs of C-G29A.2d (taken as reference) and each engineered C-G29A.2d was calculated. Table 1 shows the results. Each engineered C-G29A.2d eluted at a higher pH than C-G29A.2d from the IgG-immobilized carrier. These results suggest that carriers on which such engineered C-G29A.2d is immobilized can elute antibodies at higher pH than carriers with immobilized C-G29A.2d.

**[Table 1]**

| Ligand | Difference in elution pH (C-G29A.2d as reference) |
|---|---|
| C-F5A/G29A.2d | 0.34 |
| C-F5Y/G29A.2d | 0.11 |
| C-F13Y/G29A.2d | 0.72 |
| C-L17I/G29A.2d | 0.23 |
| C-L17I/G29A/I31L.2d | 0.57 |
| C-L17T/G29A.2d | 1.14 |
| C-L17V/G29A.2d | 0.48 |
| C-G29A/I31L.2d | 0.41 |
| C-G29A/I31US33H.2d | 0.51 |
| C-G29A/I31L/V40Q.2d | 0.59 |
| C-G29A/I31S.2d | 1.14 |
| C-G29A/I31T.2d | 0.69 |
| C-G29A/131V.2d | 0.28 |

### (Example 2) Evaluation of antibody-binding capacity of C domain variant using intermolecular interaction analyzer

The affinity of the various proteins obtained in Example 1 for immunoglobulin was analyzed using a surface plasmon resonance based biosensor "Biacore 3000" (GE Healthcare). In this example, a human immunoglobulin G preparation (hereinafter referred to as human IgG) fractionated from human plasma was used.

The human IgG was immobilized on a sensor chip, and each protein was flowed on the chip to detect an interaction between them. The immobilization of human IgG on the sensor chip CM5 was carried out by amine coupling using N-hydroxysuccinimide (NHS) and N-ethyl-N'-(3-dimethylaminopropyl)carbodiimide hydrochroride (EDC), and ethanolamine was used for blocking (the sensor chip and the immobilization reagents are all available from GE Healthcare). A human IgG solution was prepared by dissolving gammaglobulin "Nichiyaku" (Nihon Pharmaceutical Co. Ltd.) in a standard buffer (20 mM NaH₂PO₄-Na₂HPO₄, 150 mM NaCl, pH 7.4) to a concentration of 1.0 mg/mL. The human IgG solution was diluted by a factor of 100 in an immobilization buffer (10 mM CH₃COOH-CH₃COONa, pH 5.0), and the human IgG was immobilized onto the sensor chip in accordance with the protocol attached to the Biacore 3000. A reference cell as a negative control was also prepared by immobilizing ethanolamine onto another flow cell on the chip after activation with EDC/NHS.

Each protein was appropriately prepared at concentrations of 10 to 1,000 nM using running buffer (20 mM NaH₂PO₄-Na₂HPO₄, 150 mM NaCl, 0.005% P-20, pH 7.4) (three solutions with different protein concentrations were prepared for each protein), and each protein solution was added to the sensor chip at a flow rate of 20 µL/min for 30 seconds. Binding sensorgrams were sequentially measured at 25°C during the addition (association phase, 30 seconds) and after the addition (dissociation phase, 60 seconds). After each measurement, the sensor chip was regenerated for 30 seconds by adding 10 mM glycine-HCl (pH 3.0, GE Healthcare). This process was intended to remove the added proteins remaining on the sensor chip, and it was confirmed that the binding activity of the immobilized human IgG was substantially completely recovered.

The binding sensorgrams (from which the binding sensorgram of the reference cell was subtracted) were subjected to fitting using the 1:1 binding model in software BIA evaluation attached to the system to calculate the association rate constant (kₒₙ), dissociation rate constant (k_{off}), and association constant (K_{A} = k_{on/}k_{off}). Table 2 shows the results.

As shown in Table 2, the binding parameters of each engineered C-G29A.2d to human IgG were comparable to those of C-G29A.2d (control). Specifically, each ligand had an association constant with human IgG of 10⁸ M⁻¹ or more. Each engineered C-G29A.2d exhibited an antibody-binding capacity comparable to that of non-mutated C-G29A.2d in a neutral pH range.

**[Table 2]**

| Ligand | Kₒₙ(× 10⁵M⁻¹s) | K_{off}(× 10⁻³s⁻¹) | K_{A}(× 10⁹M⁻¹) |
|---|---|---|---|
| C-G29A.2d (control) | 3.9 | 0.3 | 1.2 |
| C-F5A/G29A.2d | 5.1 | 0.7 | 0.7 |
| C-F5Y/G29A.2d | 3.7 | 0.5 | 0.7 |
| C-F13Y/G29A.2d | 4.0 | 1.4 | 0.3 |
| C-L17I/G29A.2d | 4.2 | 0.5 | 0.8 |
| C-L17I/G29A/I31L2d | 5.3 | 1.0 | 0.5 |
| C-L17T/G29A.2d | 4.7 | 2.3 | 0.2 |
| C-L17V/G29A.2d | 4.6 | 0.8 | 0.6 |
| O-G29A/I31L2d | 4.7 | 0.8 | 0.6 |
| C-G29A/I31L/S33H.2d | 4.9 | 0.7 | 0.7 |
| C-G29A/I31L/V40Q.2d | 4.9 | 0.8 | 0.6 |
| C-G29A/I31S.2d | 4.6 | 2.3 | 0.2 |
| C-G29A/I31T.2d | 3.9 | 1.0 | 0.4 |
| C-G29A/I31V.2d | 4.4 | 0.5 | 0.8 |

### (Example 3) Evaluation of antibody-binding capacity of B domain variant using IgG-immobilized carrier

The total synthesis of artificially synthesized genes of engineered B-G29A.2d variants was outsourced to Eurofins Genomics K.K. These genes were synthesized by introducing amino acid substitution mutations as shown in Table 3 into a DNA (SEQ ID NO: 9) obtained by adding PstI and XbaI recognition sites to the 5' and 3' ends, respectively, of a DNA encoding B-G29A.2d (SEQ ID NO: 8) containing G29A mutation in the B domain of Protein A. Similarly to Example 1, they were recombinantly expressed, and the resulting culture supernatants were subjected to an elution test using an IgG-immobilized carrier.

The amino acid sequences of B-G29A/I31L.2d and B-G29A/I31T.2d expressed as above are shown in SEQ ID NOs: 28 and 29, respectively, in the Sequence Listing.

Table 3 shows the elution test results. Each engineered B-G29A.2d eluted at a higher pH than B-G29A.2d from the IgG-immobilized carrier. These results show that the mutations indicated in Example 1 provide similar effects on the B domain, as well as on the C domain.

**[Table 3]**

| Ligand | Difference in elution pH (B-G29A.2d as reference) |
|---|---|
| B-G29A/I31 L2d | 0.47 |
| B-G29A/I31T.2d | 0.81 |

### (Example 4) Evaluation of antibody-binding capacity of B domain variant using intermolecular interaction analyzer

The affinity of the various proteins obtained in Example 3 for immunoglobulin was analyzed as in Example 2. Table 4 shows the results. The binding parameters of each engineered B-G29A.2d to human IgG were comparable to those of B-G29A.2d (control). Each engineered B-G29A.2d exhibited an antibody-binding capacity comparable to that of non-mutated B-G29A.2d in a neutral pH range.

**[Table 4]**

| Ligand | Kₒₙ (× 10⁵M⁻¹s) | K_{off}(×10⁻³s⁻¹) | K_{A}(×10⁸M⁻¹) |
|---|---|---|---|
| B-Q29A.2d | 4.0 | 0.9 | 4.2 |
| B-G29A/I31L2d | 4.6 | 1.9 | 2.4 |
| β-G29A/I31 T.2d | 4.2 | 2.7 | 1.6 |

### (Example 5) Antibody elution test using engineered C-G29A.2d affinity separation matrix

The culture obtained as in Example 1 was centrifuged to separate the cells, and acetic acid was added to the culture supernatant to adjust the pH to 4.5, followed by standing for one hour to precipitate the target protein. The precipitate was recovered by centrifugation, and dissolved in a buffer (50 mM Tris-HCl, pH 8.5). Next, the target protein was purified by anion exchange chromatography using HiTrap Q column (GE Healthcare Bio-Sciences). Specifically, the target protein solution was added to the HiTrap Q column equilibrated with an anion exchange buffer A (50 mM Tris-HCl, pH 8.0), and washed with the anion exchange buffer A, followed by elution with a salt gradient using the anion exchange buffer A and an anion exchange buffer B (50 mM Tris-HCl, 1 M NaCl, pH 8.0) to separate the target protein eluted in the middle of the gradient. The separated target protein solution was dialyzed with ultrapure water. The dialyzed aqueous solution was used as a finally purified sample. All processes of protein purification by column chromatography were carried out using AKTA avant system (GE Healthcare Bio-Sciences).

The water-insoluble base material used was a commercially available activated prepacked column "Hitrap NHS activated HP" (1 mL) (GE Healthcare). This column is a cross-linked agarose-based column into which N-hydroxysuccinimide (NHS) groups for immobilizing proteinic ligands have been introduced. Each of the finally purified samples was immobilized as a ligand to prepare affinity separation matrices in accordance with the product manual.

Specifically, each finally purified sample was diluted to a final concentration of about 13 mg/mL in a coupling buffer (0.2 M sodium carbonate, 0.5 M NaCl, pH 8.3) to prepare a solution (1 mL). Then, 2 mL of 1 mM HCl cooled in an ice bath was flowed at a flow rate of 1 mL/min. This procedure was repeated three times to remove isopropanol from the column. Immediately thereafter, 1 mL of the sample dilution solution prepared as above was added at the same flow rate. The top and bottom of the column were sealed, and the column was left at 25°C for 30 minutes to immobilize the protein onto the column. Thereafter, the column was opened, and 3 mL of the coupling buffer was flowed at the same flow rate to recover unreacted proteins. Subsequently, 2 mL of a blocking buffer (0.5 M ethanolamine, 0.5 M NaCl, pH 8.3) was flowed. This procedure was repeated three times. Then, 2 mL of a washing buffer (0.1 M acetic acid, 0.5 M NaCl, pH 4.0) was flowed. This procedure was repeated three times. Finally, 2 mL of a standard buffer (20 mM NaH₂PO₄-Na₂HPO₄, 150 mM NaCl, pH 7.4) was flowed. Thus, the preparation of an affinity separation column was completed. An antibody elution test was performed using the affinity separation matrix under the conditions indicated below. The test was also performed using a C-G29A.2d affinity separation matrix prepared as a control in the same manner. The antibody recovery rate was calculated by measuring the absorbance of the eluate. Table 5 shows the results.

### <Conditions for antibody elution test using engineered C-G29A.2d affinity separation matrix>

Column: prepacked column "Hitrap NHS activated HP", 1 mL (GE Healthcare) (column with each ligand immobilized on carrier)
Flow rate: 0.33 mL/min; contact time: 3.0 min
Loading liquid: gammaglobulin "Nichiyaku" (Nihon Pharmaceutical Co. Ltd.), 5 mL (ligand concentration: 1 mg/mL)
Equilibration buffer: Dulbecco's phosphate buffered saline (Sigma Aldrich)

### Elution conditions:

Elution 1: 50 mM citrate buffer (4 CV); Test A: pH 4.0; Test B: pH 3.75; Test C: pH 3.5
Elution 2: 50 mM citrate buffer, pH 3.0 (4 CV)

**[Table 5]**

| | Elution pH | Antibody recovery rate (%) | | | |
|---|---|---|---|---|---|
| | | C-G29A2d (control) | C-L17I/G29A.2d | C-G29A/I31 L.2d | C-G29A/I31L/V40Q.2d |
| Test A | Elution 1 (pH 4.0) | 64 | 86 | 93 | 99 |
| | Elution 2 (pH 3.0) | 46 | 14 | 7 | 1 |
| Test B | Elution 1 (pH 3.75) | 92 | 99 | 99 | 99 |
| | Elution 2 (pH 3.0) | 8 | 1 | 1 | 1 |
| Test C | Elution 1 (pH 3.5) | 99 | 100 | 100 | 100 |
| | Elution 2 (pH 3.0) | 1 | 0 | 0 | 0 |

The affinity separation matrices prepared with each engineered C-G29A.2d exhibited higher antibody recovery rates in the eluents having a high pH (4.0 to 3.5) than the affinity separation matrix with C-G29A.2d. These results show that the ligands that had a high elution pH in the IgG Sepharose test in Example 1 exhibited a high antibody recovery rate when the antibody was eluted at a high pH using an affinity separation matrix on which each of the ligands was immobilized.

### (Example 6) Evaluation of antibody-binding capacity of C domain variant using IgG-immobilized carrier

The total synthesis of artificially synthesized genes of engineered C-G29A.2d variants was outsourced to Eurofins Genomics K.K. These genes were synthesized by introducing amino acid substitution mutations as shown in Table 6 into a DNA (SEQ ID NO: 7) obtained by adding PstI and XbaI recognition sites to the 5' and 3' ends, respectively, of a DNA encoding C-G29A.2d (SEQ ID NO: 6) containing G29A mutation in the C domain of Protein A.

The amino acid sequences of C-F5G/G29A.2d, C-F5M/G29A.2d, C-F13W/G29A.2d, C-G29A/I31F.2d, and C-G29A/I31N.2d expressed as above are shown in SEQ ID NOs: 30 to 34, respectively, in the Sequence Listing.

Similarly to Example 1, plasmids were prepared, and then recombinant cells were grown to obtain culture media containing each engineered C-G29A.2d. An elution test was performed on each engineered C-G29A.2d or C-G29A.2d in the culture supernatant using an IgG-immobilized carrier under the same conditions as in Example 1.

The difference between the elution pHs of C-G29A.2d (taken as reference) and each engineered C-G29A.2d was calculated. Table 6 shows the results. Each engineered C-G29A.2d eluted at a higher pH than C-G29A.2d from the IgG-immobilized carrier. These results suggest that carriers on which such engineered C-G29A.2d is immobilized can elute antibodies at higher pH than carriers with immobilized C-G29A.2d.

**[Table 6]**

| Ligand | Difference in elution pH (C-G29A.2d as reference) |
|---|---|
| C-F5G/G29A.2d | 0.42 |
| C-F5M/G29A.2d | 0.19 |
| C-F13W/G29A.2d | 0.48 |
| C-G29A/I31 F.2d | 0.41 |
| C-G29A/I31 N.2d | 0.90 |

### (Example 7) Evaluation of antibody-binding capacity of C domain variant using intermolecular interaction analyzer

The affinity of the various proteins obtained in Example 6 for immunoglobulin was analyzed under the same conditions as in Example 2 using a surface plasmon resonance based biosensor "Biacore 3000" (GE Healthcare). The immunoglobulin used was human IgG.

As shown in Table 7, the binding parameters of each engineered C-G29A.2d to human IgG were comparable to those of C-G29A.2d (control). Specifically, each ligand had an association constant with human IgG of 10⁹M⁻¹ or more. Each engineered C-G29A.2d exhibited an antibody-binding capacity comparable to that of non-mutated C-G29A.2d in a neutral pH range.

**[Table 7]**

| Ligand | Kₒₙ(× 10⁶M⁻¹s) | K_{off}(× 10⁻³s⁻¹) | K_{A}(× 10⁹M⁻¹) |
|---|---|---|---|
| C-G29A.2d (control) | 1.0 | 0.2 | 5.3 |
| C-F5G/G29A.2d | 1.7 | 0.4 | 4.4 |
| C-F5M/G29A.2d | 1.1 | 0.6 | 1.7 |
| C-F13W/G29A.2d | 1.4 | 0.6 | 2.4 |
| C-G29A/I31F.2d | 1.6 | 0.8 | 2.1 |
| C-G29A/I31N.2d | 1.9 | 1.9 | 1.0 |

### (Example 8) Evaluation of separation behavior between antibody and host cell impurities of engineered C-G29A.2d affinity separation matrix

An antibody elution test was performed under the conditions indicated below using the affinity separation matrix with immobilized C-G29A/I31L.2d obtained in Example 5 and a CHO cell culture supernatant containing a TNF receptor-Fc fusion protein (Etanercept) (Bioceros). The elution test was also performed using a C-G29A.2d affinity separation matrix prepared as a control in the same manner. The antibody recovery rate was calculated by measuring the absorbance of the eluate. The host cell impurities (HCP) were quantified using CHO HCP ELISA kit (Cygnus). Fig. 2 shows the results obtained with the C-G29A.2d affinity separation matrix as a control. Fig. 3 shows the results obtained with the engineered C-G29A.2d affinity separation matrix.

### <Conditions for antibody elution test using engineered C-G29A.2d affinity separation matrix>

Column: Prepacked column "Hitrap NHS activated HP", 1 mL (GE Healthcare) (column with each ligand immobilized on carrier)
Flow rate: 1 mL/min (0.33 mL/min only during sample loading; contact time: 3.0 min)
Loading liquid: CHO cell culture supernatant containing TNF receptor-Fc fusion protein (Bioceros), 16.1 mL
(concentration: 0.62 mg/mL)
Equilibration buffer: Dulbecco's phosphate buffered saline (Sigma Aldrich)

### Elution conditions:

Elution 1: gradient elution with 50 mM citrate buffer (pH 6 followed by pH 3) (20 CV)
Elution 2: 50 mM citrate buffer, pH 3.0 (5 CV)

As shown in Figs. 2 and 3, the TNF receptor-Fc fusion protein was eluted at a high pH from the affinity separation matrix prepared with the engineered C-G29A.2d, as compared to the C-G29A.2d affinity separation matrix. Moreover, the amount of host cell impurities in the eluted fraction was smaller when the affinity separation matrix prepared with the engineered C-G29A.2d was used. Specifically, the HCP content in the peak top fraction was 4187 ppm for the C-G29A.2d affinity separation matrix, and was 3109 ppm for the engineered C-G29A.2d affinity separation matrix. These results suggest that the ligands that had a high elution pH in the IgG Sepharose test in Example 1 can reduce the amount of host cell impurities in the eluate as compared to the unmodified ligand.

## Claims

1. A protein, comprising
an amino acid sequence derived from any of the E, D, A, B, and C domains of Protein A of SEQ ID NOs: 1 to 5 in which a hydrophobic amino acid residue in an Fc binding site is substituted by a different hydrophobic amino acid residue or polar uncharged amino acid residue,
wherein the protein has a reduced antibody-binding capacity in an acidic pH range as compared to before the substitution.

2. The protein according to claim 1,
wherein the hydrophobic amino acid residue in the Fc binding site is Phe corresponding to position 5, Phe corresponding to position 13, Leu corresponding to position 17, or Ile corresponding to position 31 of the C domain.

3. The protein according to claim 1 or 2,
wherein the different hydrophobic amino acid residue is Gly, Ala, Val, Leu, Ile, Met, Phe, or Trp.

4. The protein according to claim 1 or 2,
wherein the polar uncharged amino acid residue is Ser, Thr, Gln, Asn, Tyr, or Cys.

5. The protein according to any one of claims 1 to 4,
wherein at least 90% of the following amino acid residues are retained: Gln-9, Gln-10, Tyr-14, Pro-20, Asn-21, Leu-22, Gln-26, Arg-27, Phe-30, Leu-34, Pro-38, Ser-39, Leu-45, Leu-51, Asn-52, Gln-55, and Pro-57, wherein the residue numbers indicated are for the C domain.

6. The protein according to any one of claims 1 to 5,
wherein an amino acid corresponding to position 40 of the C domain is an amino acid other than Val.

7. The protein according to any one of claims 1 to 6,
wherein the amino acid sequence further contains a substitution of a basic amino acid residue for a hydrophobic amino acid residue, an acidic amino acid residue, or a polar uncharged amino acid residue.

8. A multi-domain protein, obtained by linking at least two proteins according to any one of claims 1 to 7.

9. A DNA, encoding the protein according to any one of claims 1 to 8.

10. A vector, comprising the DNA according to claim 9.

11. A transformant, produced by transforming a host cell with the vector according to claim 10.

12. A method for producing the protein according to any one of claims 1 to 8, the method comprising using the transformant according to claim 11, or a cell-free protein synthesis system comprising the DNA according to claim 9.

13. An affinity separation matrix, comprising
the protein according to any one of claims 1 to 8 as an affinity ligand immobilized on a carrier made of a water-insoluble base material.

14. The affinity separation matrix according to claim 13,
which binds to a protein containing an immunoglobulin Fc region.

15. The affinity separation matrix according to claim 14,
wherein the protein containing an immunoglobulin Fc region is an immunoglobulin G or an immunoglobulin G derivative.

16. A method for preparing the affinity separation matrix according to any one of claims 13 to 15, the method comprising
immobilizing the protein according to any one of claims 1 to 8 as an affinity ligand onto a carrier made of a water-insoluble base material.

17. A method for purifying a protein containing an immunoglobulin Fc region, the method comprising
adsorbing a protein containing an immunoglobulin Fc region onto the affinity separation matrix according to any one of claims 13 to 15.

18. The method according to claim 17,
wherein the method comprises the following steps (a) and (b):
(a) adsorbing a liquid containing a protein containing an immunoglobulin Fc region onto the affinity separation matrix according to any one of claims 13 to 15; and
(b) bringing an eluent having a pH of 3.5 or higher into contact with the affinity separation matrix to elute the protein containing an immunoglobulin Fc region.

19. The method according to claim 18,
wherein the eluted protein containing an immunoglobulin Fc region contains a reduced amount of host cell proteins and/or of aggregates of the protein containing an immunoglobulin Fc region.


SEQUENCE LISTING

<110>  KANEKA CORPORATION

<120>  ANTIBODY-BINDING PROTEIN HAVING REDUCED ANTIBODY-BINDING CAPACITY IN
ACIDIC pH RANGE

<130>  B150469WO01

<140>  PCT/JP2016/071356

<141>  2016-07-21

<150>  JP 2015-145004

<151>  2015-07-22

<160>  34

<170>  PatentIn version 3.5

<210>  1
<211>  56
<212>  PRT
<213>  Staphylococcus aureus

<400>  1

Ala Gln His Asp Glu Ala Gln Gln Asn Ala Phe Tyr Gln Val Leu Asn
1               5                   10                  15

Met Pro Asn Leu Asn Ala Asp Gln Arg Asn Gly Phe Ile Gln Ser Leu
            20                  25                  30

Lys Asp Asp Pro Ser Gln Ser Ala Asn Val Leu Gly Glu Ala Gln Lys
        35                  40                  45

Leu Asn Asp Ser Gln Ala Pro Lys
    50                  55

<210>  2
<211>  61
<212>  PRT
<213>  Staphylococcus aureus

<400>  2

Ala Asp Ala Gln Gln Asn Lys Phe Asn Lys Asp Gln Gln Ser Ala Phe
1               5                   10                  15

Tyr Glu Ile Leu Asn Met Pro Asn Leu Asn Glu Glu Gln Arg Asn Gly
            20                  25                  30

Phe Ile Gln Ser Leu Lys Asp Asp Pro Ser Gln Ser Thr Asn Val Leu
        35                  40                  45

Gly Glu Ala Lys Lys Leu Asn Glu Ser Gln Ala Pro Lys
    50                  55                  60

<210>  3
<211>  58
<212>  PRT
<213>  Staphylococcus aureus

<400>  3

Ala Asp Asn Asn Phe Asn Lys Glu Gln Gln Asn Ala Phe Tyr Glu Ile
1               5                   10                  15

Leu Asn Met Pro Asn Leu Asn Glu Glu Gln Arg Asn Gly Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Glu Ser Gln Ala Pro Lys
    50                  55

<210>  4
<211>  58
<212>  PRT
<213>  Staphylococcus aureus

<400>  4

<210>  5
<211>  58
<212>  PRT
<213>  Staphylococcus aureus

<400>  5

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Gly Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys
    50                  55

<210>  6
<211>  116
<212>  PRT
<213>  Artificial Sequence

<220>
<223>  C domain mutant

<400>  6

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  7
<211>  359
<212>  DNA
<213>  Artificial Sequence

<220>
<223>  C domain mutant

<400>  7
ctgcagataa caaatttaac aaagaacaac aaaacgcttt ctacgaaatc ctgcacttgc      60

caaaccttac tgaagaacaa cgtaatgctt tcatccaatc cctgaaagat gatccatctg     120

tatccaaaga aattttggca gaggctaaaa aacttaacga cgctcaggcg cctaaggctg     180

ataacaaatt caataaagaa cagcaaaacg ctttttatga aatccttcac ctgccaaatc     240

ttacagaaga acaacgcaac gcattcattc aaagcttgaa ggatgaccct tccgttagca     300

aagagatcct ggctgaagca aaaaagttga atgatgcgca agcaccaaaa taatctaga      359

<210>  8
<211>  116
<212>  PRT
<213>  Artificial Sequence

<220>
<223>  B domain mutant

<400>  8

Ala Asp Asn Lys Phe Asn Lys Glu Gln Gln Asn Ala Phe Tyr Glu Ile
1               5                   10                  15

Leu His Leu Pro Asn Leu Asn Glu Glu Gln Arg Asn Ala Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Asn Glu Glu Gln Arg Asn Ala Phe Ile Gln Ser Leu Lys Asp Asp Pro
                85                  90                  95

Ser Gln Ser Ala Asn Leu Leu Ala Glu Ala Lys Lys Leu Asn Asp Ala
            100                 105                 110

Gln Ala Pro Lys
        115

<210>  9
<211>  359
<212>  DNA
<213>  Artificial Sequence

<220>
<223>  B domain mutant

<400>  9
ctgcagataa caaatttaac aaagaacaac aaaacgcttt ctacgaaatc ctgcacttgc      60

caaaccttaa tgaagaacaa cgtaatgctt tcatccaatc cctgaaagat gatccatctc 120

aatccgctaa ccttttggca gaggctaaaa aacttaacga cgctcaggcg cctaaggctg 180

ataacaaatt caataaagaa cagcaaaacg ctttttatga aatccttcac ctgccaaatc 240

ttaacgaaga acaacgcaac gcattcattc aaagcttgaa ggatgaccct tcccaaagcg 300

caaatttgct ggctgaagca aaaaagttga atgatgcgca agcaccaaaa taatctaga 359

<210> 10
<211> 116
<212> PRT
<213> Artificial Sequence

<220>
<223> C-F5A/G29A.2d

<400> 10

Ala Asp Asn Lys Ala Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Ala Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210> 11
<211> 116
<212> PRT
<213> Artificial Sequence

<220>
<223> C-F5Y/G29A.2d

<400> 11

Ala Asp Asn Lys Tyr Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Tyr Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  12
<211>  116
<212>  PRT
<213>  Artificial Sequence

<220>
<223>  C-F5G/G29A.2d

<400>  12

Ala Asp Asn Lys Gly Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Gly Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  13
<211>  116
<212>  PRT
<213>  Artificial Sequence

<220>
<223>  C-F5M/G29A.2d

<400>  13

Ala Asp Asn Lys Met Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Met Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  14
<211>  116
<212>  PRT
<213>  Artificial Sequence

<220>
<223>  C-F13Y/G29A.2d

<400>  14

Ala Asp Asn Lys Phe Asn Lys Glu Gln Gln Asn Ala Tyr Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Tyr Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  15
<211>  116
<212>  PRT
<213>  Artificial Sequence

<220>
<223>  C-F13W/G29A.2d

<400>  15

Ala Asp Asn Lys Phe Asn Lys Glu Gln Gln Asn Ala Trp Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Trp Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  16
<211>  116
<212>  PRT
<213>  Artificial Sequence

<220>
<223>  C-L17I/G29A.2d

<400>  16

Ala Asp Asn Lys Phe Asn Lys Glu Gln Gln Asn Ala Phe Tyr Glu Ile
1               5                   10                  15

Ile His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Ile His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  17
<211>  116
<212>  PRT
<213>  Artificial Sequence

<220>
<223>  C-L17I/G29A/I31L.2d

<400>  17

Ala Asp Asn Lys Phe Asn Lys Glu Gln Gln Asn Ala Phe Tyr Glu Ile
1               5                   10                  15

Ile His Leu Pro Asn Leu Thr Glu Glu Gln Arg Asn Ala Phe Leu Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Ile His Leu Pro Asn Leu
65                  70                  75                  80

Thr Glu Glu Gln Arg Asn Ala Phe Leu Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  18
<211>  116
<212>  PRT
<213>  Artificial Sequence

<220>
<223>  C-L17T/G29A.2d

<400>  18

Ala Asp Asn Lys Phe Asn Lys Glu Gln Gln Asn Ala Phe Tyr Glu Ile
1               5                   10                  15

Thr His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Thr His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  19
<211>  116
<212>  PRT
<213>  Artificial Sequence

<220>
<223>  C-L17V/G29A.2d

<400>  19

Ala Asp Asn Lys Phe Asn Lys Glu Gln Gln Asn Ala Phe Tyr Glu Ile
1               5                   10                  15

Val His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Val His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  20
<211>  116
<212>  PRT
<213>  Artificial Sequence

<220>
<223>  C-G29A/I31L.2d

<400>  20

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Leu Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Leu Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  21
<211>  116
<212>  PRT
<213>  Artificial Sequence

<220>
<223>  C-G29A/I31F.2d

<400>  21

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Phe Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Phe Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  22
<211>  116
<212>  PRT
<213>  Artificial Sequence

<220>
<223>  C-G29A/I31N.2d

<400>  22

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Asn Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Asn Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  23
<211>  116
<212>  PRT
<213>  Artificial Sequence

<220>
<223>  C-G29A/I31L/S33H.2d

<400>  23

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Leu Gln
            20                  25                  30

His Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Leu Gln His Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  24
<211>  116
<212>  PRT
<213>  Artificial Sequence

<220>
<223>  C-G29A/I31L/V40Q.2d

<400>  24

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Leu Gln
            20                  25                  30

Ser Leu Lys Asp Asp Pro Ser Gln Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Leu Gln Ser Leu Lys Asp Asp Pro
                85                  90                  95

Ser Gln Ser Lys Glu Ile Leu Ala Glu Ala Lys Lys Leu Asn Asp Ala
            100                 105                 110

Gln Ala Pro Lys
        115

<210>  25
<211>  116
<212>  PRT
<213>  Artificial Sequence

<220>
<223>  C-G29A/I31S.2d

<400>  25

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Ser Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ser Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  26
<211>  116
<212>  PRT
<213>  Artificial Sequence

<220>
<223>  C-G29A/I31T.2d

<400>  26

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Thr Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Thr Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  27
<211>  116
<212>  PRT
<213>  Artificial Sequence

<220>
<223>  C-G29A/I31V.2d

<400>  27

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Val Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Val Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  28
<211>  116
<212>  PRT
<213>  Artificial Sequence

<220>
<223>  B-G29A/I31L.2d

<400>  28

Ala Asp Asn Lys Phe Asn Lys Glu Gln Gln Asn Ala Phe Tyr Glu Ile
1               5                   10                  15

Leu His Leu Pro Asn Leu Asn Glu Glu Gln Arg Asn Ala Phe Leu Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Asn Glu Glu Gln Arg Asn Ala Phe Leu Gln Ser Leu Lys Asp Asp Pro
                85                  90                  95

Ser Gln Ser Ala Asn Leu Leu Ala Glu Ala Lys Lys Leu Asn Asp Ala
            100                 105                 110

Gln Ala Pro Lys
        115

<210>  29
<211>  116
<212>  PRT
<213>  Artificial Sequence

<220>
<223>  B-G29A/I31T.2d

<400>  29

Ala Asp Asn Lys Phe Asn Lys Glu Gln Gln Asn Ala Phe Tyr Glu Ile
1               5                   10                  15

Leu His Leu Pro Asn Leu Asn Glu Glu Gln Arg Asn Ala Phe Thr Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Asn Glu Glu Gln Arg Asn Ala Phe Thr Gln Ser Leu Lys Asp Asp Pro
                85                  90                  95

Ser Gln Ser Ala Asn Leu Leu Ala Glu Ala Lys Lys Leu Asn Asp Ala
            100                 105                 110

Gln Ala Pro Lys
        115

<210>  30
<211>  116
<212>  PRT
<213>  Artificial Sequence

<220>
<223>  C-F5G/G29A.2d

<400>  30

Ala Asp Asn Lys Gly Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Gly Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  31
<211>  116
<212>  PRT
<213>  Artificial Sequence

<220>
<223>  C-F5M/G29A.2d

<400>  31

Ala Asp Asn Lys Met Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Met Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  32
<211>  116
<212>  PRT
<213>  Artificial Sequence

<220>
<223>  C-F13W/G29A.2d

<400>  32

Ala Asp Asn Lys Phe Asn Lys Glu Gln Gln Asn Ala Trp Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Trp Tyr Glu Ile Leu His Leu Pro Asn Leu
65                  70                  75                  80

Thr Glu Glu Gln Arg Asn Ala Phe Ile Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  33
<211>  116
<212>  PRT
<213>  Artificial Sequence

<220>
<223>  C-G29A/I31F.2d

<400>  33

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Phe Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Phe Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115

<210>  34
<211>  116
<212>  PRT
<213>  Artificial Sequence

<220>
<223>  C-G29A/I31N.2d

<400>  34

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Asn Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys Ala Asp Asn Lys Phe Asn
    50                  55                  60

Lys Glu Gln Gln Asn Ala Phe Tyr Glu Ile Leu His Leu Pro Asn Leu
65                  70                  75                  80

Thr Glu Glu Gln Arg Asn Ala Phe Asn Gln Ser Leu Lys Asp Asp Pro
                85                  90                  95

Ser Val Ser Lys Glu Ile Leu Ala Glu Ala Lys Lys Leu Asn Asp Ala
            100                 105                 110

Gln Ala Pro Lys
        115